# EUROPÄISCHE PATENTANMELDUNG

(11) **EP 3 081 220 A1**
(43) Veröffentlichungstag der Anmeldung: **19.10.2016**
(21) Anmeldenummer: 16161494.6
(22) Anmeldetag: 22.03.2016
(51) Int. Cl.: A61K 35/16, A61K 9/00, A61J 1/05, A61M 1/02, A61P 27/04

(54) **VERFAHREN ZUR HERSTELLUNG VON AUGENTROPFEN**

(30) Priorität: 24.03.2015 DE 102015205293
(71) Anmelder: Jochheim-Richter, Andrea, 61462 Koenigstein (DE)
(72) Erfinder: JOCHHEIM-RICHTER, Andrea, 61462 Koenigstein (DE); SCHROTT, Marc, 61449 Steinbach (DE)
(74) Vertreter: Schiweck, Weinzierl & Koch

(57) **Zusammenfassung**

Die vorliegende Erfindung betrifft ein Verfahren zur Herstellung von Augentropfen, die so erhaltenden Augentropfen sowie ein Kit, welches die Augentropfen umfasst.

## Beschreibung

Die vorliegende Erfindung betrifft ein Verfahren zur Herstellung von Augentropfen, die so erhaltenen Augentropfen sowie ein Kit, welches die Augentropfen umfasst.

Das trockene Auge ist eine der häufigsten Erkrankungen im Bereich der Augenheilkunde, wobei die Prävalenz mit zunehmendem Alter ansteigt. Das trockene Auge ist, gemäß der Definition der "Dry Eye Workshop Study Group" (DEWS Steering Committee), eine multifaktorielle Störung von Tränenfilm und Augenoberfläche, welche mit physischpsychischem Unbehagen oder visueller Beeinträchtigung einhergeht. Inflammatorische Prozesse und ein hyperosmolarer Tränenfilm sind wichtige Faktoren, die mit diesem Krankheitsbild assoziiert werden.

Unter dem Krankheitsbild des trockenen Auges wird häufig eine Keratokonjunktivitis sicca verstanden. Die Keratokonjunktivitis sicca bezeichnet eine Augenoberflächenerkrankung, welche mit typischen Symptomen wie Fremdkörpergefühl, Lichtempfindlichkeit, Juckreiz, brennenden Schmerzen und Druckgefühl einhergeht. Außerdem werden oft eine Visusminderung, gerötete Augen, Epiphora (Tränenträufeln) und das Gefühl des trockenen Auges beobachtet. Bei verminderter Tränenproduktion treten die Beschwerden bevorzugt morgens auf. Liegt das Problem in der vermehrten Verdunstung der Tränenflüssigkeit werden die Symptome häufiger abends beklagt.

Zur Behandlung des trockenen Auges werden heutzutage eine Vielzahl von künstlichen Tränenersatzmitteln in Form von Augentropfen, Augengel oder Augenspray angeboten. Beispielsweise sind hier der Ersatz oder die Ergänzung der wässrigen Phase der Tränenflüssigkeit durch wässrige Lösungen von Verdickungsmitteln, wie beispielsweise Povidon, Hyproxymellose oder Carboxymethylcellulose, Salzen und gegebenenfalls weiteren Wirkstoffen, wie Hyaluronsäure oder Lipiden, zu nennen. Bekannt ist auch die Ergänzung der oberflächlichen Lipidschicht des Tränenfilms durch ein liposomales Augenspray, wodurch die Verdunstung der natürlichen Tränenflüssigkeit reduziert und ein frühzeitiges Ablaufen als Träne über den Lidrand vermieden wird. Bekannt ist weiterhin der Satz des Tränenfilms und die Verringerung der Scherkräfte zwischen Augenoberfläche und Lied bei den Liedbewegungen durch fetthaltige Salben. Bei diesen Salben handelt es sich insbesondere um Öl in Wasser-Emulsionen.

Der Nachteil dieser bekannten Produkte ist, dass sie eine relative kurze Verweilzeit auf der Hornhaut haben. Dadurch bedingt muss das entsprechende Behandlungsmittel in regelmäßigen Abständen appliziert werden, was für den Patienten häufig unangenehm und in manchen Situationen unmöglich ist. Bei einem ausgeprägt trockenen Auge sollten beispielsweise alle 30 bis 60 Minuten jeweils 1 Tropfen einer wässrigen Phase in jedes Auge getropft werden, um bestehende Defizite auszugleichen. Bei gelförmigen Behandlungsmitteln wird dieser Nachteil zumindest teilweise vermieden. Es erweist sich hier jedoch schwierig, die Hornhaut ausreichend mit Sauerstoff zu versorgen. Zudem kann, ebenso wie bei Salben, für einige Sekunden bis hin zu Minuten die Sicht beeinträchtigt werden. Umfassen die entsprechenden Mittel Konservierungsstoffe, können diese zum einen zu allergischen Reaktionen führen. Bei dauerhafter häufiger Anwendung kann dies zudem zu einer Verschlechterung der Symptomatik des trockenen Auges führen.

Bekannt ist weiterhin die Verwendung von Punctum Plaques. Bei der Ableitung der von den Tränendrüsen gebildeten Tränenflüssigkeit zur Nase wird die Tränenflüssigkeit zunächst über den Liedschlag über die Hornhaut verteilt, um diese feucht zu erhalten. Anschließend wird sie über die Tränenpunkte (Puncta lacrimalia) aus dem Bindehautsack abgeleitet. Punctum Plaques werden in ein oder beide Puncta lacrimalia eingesetzt, so dass dieser Abfluss reduziert ist. Die Plaques können je nach Ausführung dauerhaft in der Puncta lacrimalia verbleiben oder sich nach ein paar Wochen auflösen. Der wesentliche Nachteil dieses Verfahrens ist, dass durch das Rückhalten der Tränenflüssigkeit gleichzeitig auch die entzündungsfördernden Stoffe in der Tränenflüssigkeit, die beim trockenen Auge eine veränderte Zusammensetzung aufweist, länger am Auge verbleiben. Der durch die Plaques verringerte Abfluss von Tränenflüssigkeit durch die ableitenden Tränenwege kann zudem Infektionen durch Bakterien, die aus dem Nasenraum aufsteigen können, Vorschub leisten.

Insbesondere bei ausgeprägten Augenoberflächenerkrankungen hat sich gezeigt, dass die bekannten künstlichen Tränenersatzmittel oder der Einsatz von Punctum Plaques nicht immer zum gewünschten Erfolg führen. Die künstlichen Tränenersatzmittel enthalten immer nur wenige Bestandteile der natürlichen Tränen. Eine nachhaltige Therapie ist hier schwer möglich.

Ein relativ neuer Ansatz in der Therapie von Erkrankungen der Augenoberfläche ist der Einsatz von Augentropfen aus Serum, insbesondere aus Eigenserum. Vorteilhaft daran ist, dass entsprechende Augentropfen eine biomechanische und biochemische Ähnlichkeit zur Tränenflüssigkeit aufweisen. Die Therapieerfolge sind hier besser als mit sonst üblicherweise eingesetzten künstlichen Tränenersatzmitteln, wie von E.M. Herold ("Therapie der Keratokonjunktivitis Sicca mit Augentropfen aus autologem Serum", Inaugural-Dissertation Universität Erlangen Nürnberg, 20. Dezember 2012) gezeigt wurde. Eigenserum bedeutet autologes Serum, das aus Blut der Person, die später die hieraus hergestellten Augentropfen einsetzt, gewonnen wurde.

Ein mögliches Verfahren zur Herstellung von Eigenserumaugentropfen ist beispielsweise von Herold (a.a.o.) beschrieben. Hier werden vom Patienten 100 ml Eigenblut entnommen. Das Blutbild wird vor und nach der Entnahme kontrolliert. Nach Zentrifugation wird der Überstand steril abpipettiert und in sterile Tropfflaschen gegeben. Unmittelbar danach werden die Tropffläschchen bei mindestens -18°C eingefroren. Aufgetaute Flaschen sollten bei +2°C bis +6°C maximal eine Woche gelagert werden.

Der hohe Anteil an Proteinen im Serum bedingt eine mikrobielle Anfälligkeit. Der Einsatz von Stabilisatoren oder Konservierungsmitteln ist jedoch, ebenso wie eine Pasteurisierung nicht möglich beziehungsweise nicht erwünscht. Durch den Verzicht auf Konservierungsstoffe beziehungsweise Antibiotika in entsprechenden Eigenserumaugentropfen besteht die potenzielle Gefahr einer bakteriellen Kontamination, welche insbesondere bei längerer Verwendung der Augentropfen aus demselben Dosiergefäß beziehungsweise Verabreichungsbehältnis besteht. Zudem ist die Behandlung der Keratokonjunktivitis sicca üblicherweise auf einen Zeitraum von 6 Monaten oder länger angelegt. Eine Entnahme von 100 ml Eigenblut, wie aus dem Stand der Technik bekannt, ist jedoch nicht ausreichend, um die Behandlung über einen längeren Zeitraum auszuführen. Da die Blutabnahme von dem betroffenen Menschen üblicherweise als unangenehm empfunden wird, besteht zudem Bedarf an einem Herstellungsverfahren, welches zu einer höheren Compliance und damit zum Erfolg der entsprechenden Behandlung beiträgt. Zudem sollten auch die weiteren Nachteile aus dem Stand der Technik möglichst vermieden werden.

Überraschenderweise hat sich gezeigt, dass die der vorliegenden Erfindung zugrunde liegende Aufgabe gelöst wird durch ein Verfahren zur Herstellung von gebrauchsfertigen Augentropfen, insbesondere zur Behandlung des trockenen Auges, welches die folgenden Schritte umfasst:
a) Entnahme von Vollblut an einem ersten Ort und anschließende
b) Auftrennung des Blutes zum Erhalt von Serum,
c) Untersuchung des Serums auf das Vorliegen von Pathogenen gefolgt von
d) Abfüllung des Serums in wenigstens ein Verabreichungsmittel an einem zweiten Ort,
wobei erster Ort und zweiter Ort voneinander verschieden sind.

Bevorzugt umfasst das Verfahren weiterhin Schritt
e) Abschlussuntersuchungen vor der Freigabe der Augentropfen als Arzneimittel auf Sterilität.

Bei der Abschlussuntersuchung können gegebenenfalls auch Identität und/oder Reinheit zusätzlich überprüft werden.

Gebrauchsfertige Augentropfen im Sinne der vorliegenden Erfindung sind solche Augentropfen, die von Personen unmittelbar eingesetzt werden können. Die Abgabe von Serum-Augentropfen ist ein Apothekengebundener Vorgang. Die Abfüllung des Serums in ein Verabreichungsmittel erfolgt in dem erfindungsgemäßen Verfahren in der Apotheke. Die Blutentnahme selbst erfolgt an einem anderen Ort. Dies ist beispielsweise mit einer Vorrichtung, wie sie in WO 2010/136535 A1 beschrieben ist, nicht möglich, da der Blutentnahmebeutel unmittelbar mit dem Verabreichungsmittel verbunden ist. Die Durchführung der Blutentnahme an einem Ort, beispielsweise beim Blutspendedienst, und die Abfüllung des Serums an einem zweiten Ort, nämlich gemäß der gesetzlichen Vorschrift in einer Apotheke, ermöglichen die Abgabe der Augentropfen in einer Apotheke beispielsweise in der Nähe des Wohnortes oder in der Nähe des Arbeitsplatzes des Anwenders.

Die Entnahme von größeren Blutmengen als 100 ml oder mehr als 200 ml, zum Beispiel von 500 ml, erfolgt vorzugsweise im Rahmen einer Vollblutspende. Unter Blut beziehungsweise Vollblut ist das Blut zu verstehen, welches sämtliche nativen Bestandteile enthält. Die Vollblutspende kann nach Standardverfahren von einem Transfusionsmediziener beispielsweise in entsprechenden Blutspendezentalen entnommen werden. Eine Vollblutspende, bei der etwa 500 ml Blut entnommen werden ermöglicht nach dessen Auftrennung den Erhalt von etwa 230 ml Serum. Aus diesem Serum können dann Augentropfen gewonnen werden, die eine Versorgung einer Person, welche unter trockenen Augen leidet, über einen Zeitraum von bis zu einem halben Jahr ermöglichen. Das erfindungsgemäße Verfahren ermöglicht somit eine langfristige Behandlung, ohne dass eine weitere Blutentnahme notwendig ist, wodurch eine verbesserte Compliance und damit auch bessere Behandlungserfolge gegeben sind.

Die Entnahme von 500 ml Blut entspricht der Menge an Blut, welche im Rahmen einer Vollblutspende üblicherweise entnommen wird. Die Entnahme von mehr als 500 ml Blut und die Weiterverarbeitung zu Augentropfen aus Eigenserum ist grundsätzlich möglich. Aus medizinischer Sicht wird jedoch empfohlen, eine Blutspende auf eine Menge von 500 ml zu begrenzen. Diese Entnahmemenge ist für den menschlichen Organismus unschädlich. Erfindungsgemäß ist es auch möglich weniger als 500 ml Blut, wie beispielsweise 300 ml, 350 ml, 400 ml oder 450 ml, zu entnehmen. Auch hier ist eine längerfristige Versorgung von Personen mit Serumaugentropfen noch möglich. Die Entnahme von weniger als 200 ml Blut ist jedoch nicht zu empfehlen, da hier eine häufigere Blutentnahme notwendig ist, wodurch die Compliance und damit der Behandlungserfolg herabgesetzt werden.

Erfindungsgemäß kann es sich bei dem Vollblut um autologes oder allogenes Vollblut handeln. Autologes Vollblut bedeutet, dass die aus dem Serum gewonnenen Augentropfen ausschließlich von der Person eingesetzt werden, die auch das Blut gespendet hat. Im Fall von allogenem Vollblut sind Blutspender und Verwender der Augentropfen unterschiedliche Personen.

Der Einsatz von autologem Vollblut hat den Vorteil, dass nur körpereigene Stoffe in den dann erhaltenen Eigenserumaugentropfen vorhanden sind. Sind Personen beispielsweise auf Grund gesundheitlicher Einschränkungen nicht in der Lage selbst Blut zu spenden, so ermöglicht eine allogene Blutspende dennoch die Versorgung dieser Personen mit Serumaugentropfen. Allogenes Vollblut ermöglicht hier somit eine Behandlung, welche mit autologem Vollblut nicht möglich wäre, da dieses nicht zur Verfügung steht.

Auf Grund der mehrfachen Überprüfungsmöglichkeiten des Vollblutes sowie des Serums im erfindungsgemäßen Verfahren kann die Compliance von Serumaugentropfen aus allogenem Vollblut verbessert werden und die Sicherheit des Produktes im Sinne von Abwesenheit von Kontaminanten erhöht werden.

Die Auftrennung des Blutes in seine Blutbestandteile erfolgt durch Koagulation. Diese geschieht vorzugsweise über einen Zeitraum von 15 Minuten bis 70 Minuten bei Raumtemperatur. Raumtemperatur im Sinne der vorliegenden Erfindung ist eine Temperatur im Bereich von 15 °C bis 30 °C, insbesondere von 18 °C bis 25 °C. Unter Koagulation versteht man die Gerinnung von Blut. Hierdurch kommt es zu einer Auftrennung der Blutbestandteile. Nach der Koagulation ist es erfindungsgemäß möglich, das Blut bei +2°C bis +8°C zu lagern. Die Lagerung kann beispielsweise über Nacht erfolgen sollte jedoch ein Zeitraum von 48 Stunden auf Grund der mikrobiellen Anfälligkeit des Serums nicht überschreiten. Zudem beginnt nach etwa 48 Stunden ein enzymatischer Abbau der Blutkomponeten. Andererseits ist eine Lagerung von 12 bis 48 Stunden bevorzugt, da hier sicher gestellt werden kann, dass die Koagulation vollständig abgeschlossen ist.

Im Anschluss an die Koagulation erfolgt, gegebenenfalls nach einer Zwischenlagerung, vorzugsweise eine Zentrifugation des durch die Koagulation bereits wenigstens teilweise aufgetrennten Blutes. Die Zentrifugation erfolgt dabei vorzugsweise über einen Zeitraum von 5 bis 20 Minuten, insbesondere von 5 bis 15 Minuten, bei 1000 bis 3500 Umdrehungen/min, insbesondere bei 1000 bis 3000 Umdrehungen/min. Bei Raumtemperatur erfolgt dann die Überführung des so gewonnenen zellfreien Serums aus der ursprünglichen Blutentnahmevorrichtung, beispielsweise einem Blutbeutel, in einen für Serum geeigneten Behälter. Hierbei handelt es sich bevorzugt um einen leeren Blutbeutel (Leerbeutel, Serumbeutel), der über einen Verbindungsschlauch an den das aufgetrennte Blut enthaltenden Blutbeutel angeschweißt werden kann. Hierdurch kann eine Kontamination des Serums durch äußere Einflüsse nahezu vermieden werden.

Durch die Auftrennung des Blutes, welche bevorzugt mittels Koagulation und Zentrifugation erfolgt, erhält man somit Serum. Die physiologische Serumosmolarität (Isotonie) beträgt beim Menschen üblicherweise 281 bis 297 mosmol/l. Dieser Wert entspricht dem von Tränenflüssigkeit, wodurch sich der bevorzugte Einsatz von Eigenserum Augentropfen bei der Behandlung von Augenerkrankungen und insbesondere vom Trockenen Auge ergibt. Weiterhin liegt auch der pH Wert sowohl von Serum als auch von menschlichen Tränen bei etwa 7,4.

Das gewonnene Serum wird vor der Abfüllung in geeignete Verabreichungsmittel auf das Vorhandensein von Pathogenen untersucht. Pathogene im Sinne der vorliegenden Erfindung sind jede Art von infektiösen Erregern oder anderer Erkrankungen auslösende oder begünstigende Faktoren, die sich nachteilig auf den menschlichen Organismus auswirken können und insbesondere bei der Verwendung als Augentropfen den Therapieerfolg gefährden könnten oder möglicherweise zur Ausbildung weiterer Krankheiten führen könnten. Kontaminationen des Serums können beispielsweise auch während der Blutentnahme oder der Auftrennung des Blutes erfolgt sein. Insbesondere ist das Einbringen von Keimen bei unsachgemäßer Hautdesinfektion und/oder bei einer Leckage im Blutbeutel-Schlauchsystem möglich.

Zusätzlich zu der Untersuchung des Serums kann erfindungsgemäß auch bereits vor der Blutabnahme eine entsprechende Untersuchung des Blutes erfolgen. Dies ist auch empfehlenswert, da bei Vorliegen von Kontaminanten das Blut zur Herstellung von Augentropfen verworfen werden kann.

Auch wenn vor der Abnahme eine Analyse des Blutes durchgeführt wurde, stellt die erfindungsgemäße erneute Untersuchung des Serums eine zusätzliche Sicherheit da, um eine verbesserte Produktsicherheit zu gewährleisten. Dabei ist es erfindungsgemäß möglich, das Serum vor der Abfüllung in ein Verabreichungsmittel bei einer Temperatur von - 20°C zu lagern. Die Untersuchung auf Kontaminanten erfolgt in diesem Fall bevorzugt nach der Lagerung und unmittelbar vor der Abfüllung in das Verabreichungsmittel.

Erfindungsgemäß wird das Serum insbesondere bis zum Vorliegen der Befunde der Infektionsuntersuchung in einem temperaturkontrollierten Kühlschrank zwischengelagert. Sind die Infektionstests negativ ausgefallen, können die entsprechenden Chargen in geeignete Verabreichungsmittel abgefüllt werden.

Bei der Abfüllung des Serums in Schritt d) des erfindungsgemäßen Verfahrens sollte darauf geachtet werden, dass hierbei keine Kontamination des Serums erfolgt. Daher geschieht die Abfüllung in Schritt d) vorzugsweise in einem Reinraum. Dabei kann sich der Reinraum in der Nähe des Ortes befinden, an dem die Vollblutspende erfolgt ist. Es ist erfindungsgemäß jedoch auch möglich, dass das Serum oder sogar das Vollblut zunächst an einen anderen Ort transportiert wird und dann an diesem räumlich getrennten anderen Ort die Aufarbeitung zum Serum und/oder die Abfüllung in Verabreichungsmittel erfolgt. Dabei ist zu beachten, dass während des Transports die Temperatur von 10°C insbesondere von 8°C nicht überschritten wird.

Gegenüber dem aus dem Stand der Technik bekannten Verfahren, wie es beispielsweise in WO 2010/136535 A1 beschrieben ist, besteht erfindungsgemäß somit ein weiterer Vorteil darin, dass das erfindungsgemäße Verfahren eine ortsunabhängige Herstellung von Eigenserumaugentropfen ermöglicht, was die flexiblere Anwendung des erfindungsgemäßen Verfahrens bedingt. Ist für Blutentnahme und Erhalt der Augentropfen eine weite Wegstrecke für den Endverbraucher zurückzulegen, so führt auch dies bei ihm oft zur einer geringeren Compliance. Mit dem erfindungsgemäßen Verfahren ist es nun möglich, dass eine Vollblutspende beispielsweise bei einem Transfusionsmediziner erfolgt. Auch wenn die hierfür für den Blutspender zurück zulegende Wegstrecke einen gewissen Aufwand bedeutet, so ist dies auf Grund der niedrigeren Frequenz von voraussichtlich zweimal jährlich zumutbar. Auch hierdurch werden die Compliance und damit der Behandlungserfolg unterstützt.

Mit Hilfe des erfindungsgemäßen Verfahrens ist es daher möglich, dass die Abgabe der Eigenserumaugentropfen an den Patienten in der Nähe des Wohnortes erfolgt. Liegt der Ort der Blutentnahme mehrere Kilometer vom Wohnort des Patienten entfernt, so besteht die Belastung für den Patienten lediglich darin, einmal in 6 Monaten dorthin zu fahren. Nach der Auftrennung des Blutes ermöglicht das erfindungsgemäße Verfahren den Transport des Serums beispielsweise in eine Apotheke, welche vorzugsweise einen Reinraum aufweist. In dieser Apotheke kann das Serum dann abgefüllt und in Einzeldosen an den Patienten abgegeben werden. Alternativ ist es auch möglich, dass sich der Reinraum in der Nähe des Ortes befindet, an welchem die Blutabnahme erfolgt. Hier kann nach Abfüllung in Verabreichungsmittel der Transport zu einer wohnortnahen Apotheke erfolgen. Das erfindungsgemäße Verfahren ermöglicht somit eine äußerst hohe Flexibilität, die zu einer wohnortnahen Versorgung des Patienten mit Eigenserumaugentropfen führt.

Bevorzugt umfasst das Verfahren daher die folgenden Schritte:
a) Entnahme von Vollblut in einem Blutbeutel an einem ersten Ort,
b) Auftrennung des Blutes zum Erhalt von Serum an dem ersten Ort,
   b1) Überführung des Serums unter sterilen Bedingungen in einen Serumbeutel an dem ersten Ort,
c) Untersuchung des Serums auf das Vorliegen von Pathogenen am ersten Ort und gleichzeitiger Transport des Serums zu einem zweiten Ort,
d) Abfüllung des Serums in wenigstens ein Verabreichungsmittel am zweiten Ort.

Alternativ und ebenso bevorzugt umfasst das Verfahren die folgenden Schritte:
a) Entnahme von Vollblut in einem Blutbeutel an einem ersten Ort und anschließender Transport des Vollblutes zu einem zweiten Ort,
b) Auftrennung des Blutes zum Erhalt von Serum an dem zweiten Ort,
   b1) Überführung des Serums unter sterilen Bedingungen in einen Serumbeutel an dem zweiten Ort,
c) Untersuchung des Serums auf das Vorliegen von Pathogenen am zweiten Ort und
d) Abfüllung des Serums in wenigstens ein Verabreichungsmittel am zweiten Ort.

Die Abfüllung des Serums in Verabreichungsmittel kann in einfacher Weise zum Beispiel mittels eines Dreiwegehahns erfolgen. Der Dreiwegehahn wird dabei mit einem Anschluss an einen Beutel, in dem sich das Serum befindet (Serumbeutel) angeschlossen. Entsprechende Beutel weisen üblicherweise einen Schlauch auf, an den der Dreiwegehahn angeschlossen werden kann. An einen weiteren Anschluss des Dreiwegehahns kann dann das Verabreichungsmittel angeschlossen werden. Über die Stellung des Hahnes wird dann die Abfüllung des Serums in das Verabreichungsmittel gesteuert. Abhängig vom Volumen des Serums und dem Volumen des Verabreichungsmittels ist es möglich an die dritte Anschlussstelle des Dreiwegehahns entweder einen weiteren Serumbeutel anzuschließen, um eine vollständige Befüllung des Verabreichungsmittels zu ermöglichen. Dies ist jedoch nicht bevorzugt, da es hier zu Kreuzkontaminationen zwischen den Serum-Batches kommen kann. Bevorzugt wird an den Dreiwegehahn ein weiteres Verabreichungsbehältnis angeschlossen. Das erfindungsgemäße Verfahren ermöglicht den Erhalt eines großen Volumens an Serum. Ist es nicht möglich, das gesamte Serum in ein Verabreichungsmittel zu füllen, müsste an den Serumbeutel ein neues Verabreichungsmittel angeschlossen werden. Durch den Einsatz des Dreiwegehahnes kann dieser Wechsel vermieden werden und das gesamte Volumen an Serum kann in geeignete Verabreichungsmittel abgefüllt werden.

Dabei erfolgt die Abfüllung vorzugsweise in einem Reinraum. Hierdurch wird eine weitere Infektion des Serums vermieden. Bevorzugt Verabreichungsmittel im Sinne der vorliegenden Erfindung sind Verabreichungsbehältnisse.

Geeignete Verabreichungsbehältnisse sind aus dem Stand der Technik bekannt. Ein mögliches Verabreichungsbehältnis, welches in DE 20 2011 004 487 U1 beschrieben wird, umfasst einen Auffang- und Entlüftungsbehälter, der über eine Endleitung mit den Verabreichungsbehältnissen verbunden ist, wobei die Verabreichungsbehältnisse jeweils mit Öffnungsmitteln versehen sind und durch strömen miteinander verbunden sind. Die Verabreichungsbehältnisse können mittels eines sterilen Schlauchstücks an den Serumbeutel beispielsweise mit Hilfe eines Dreiwegehahns angeschlossen werden. Die einzelnen Verabreichungsbehältnisse haben jeweils ein Volumen von etwa 3 ml.

Nachteilig ist hier, dass das Schlauchsystem, mit welchem die Verabreichungsbehältnisse untereinander verbunden sind, ein Volumen aufweist, in welchem sich Serum sammelt, das nicht zur Verwendung als Eigenserumaugentropfen zur Verfügung steht (Totvolumen). Die einzelnen Verabreichungsbehältnisse sind zudem aus einem nachgiebigen oder weichen Material hergestellt. Je nach ausgeübtem Druck werden unterschiedliche Tropfenmengen abgegeben. Die Volumina, die für die Therapie zur Anwendung kommen und der daraus abzuleitenden Therapiedauer sind nicht vorhersehbar. Zudem birgt die große Öffnung ein hohes Risiko der Kontamination des Flascheninhalts beispielweise mit Bakterien oder Pilzen. Sobald die Einmaldosierungen geöffnet sind, können sie nicht wieder verschlossen werden, so dass die in jedem Verabreichungsbehältnis zur Verfügung stehenden Augentropfen innerhalb weniger Stunden verbraucht werden oder auf Grund der Gefahr der Kontamination verworfen werden müssen. Auf Grund des großen Totvolumens und sowie der extrem kurzen Haltbarkeit der einzelnen Verabreichungsbehältnisse, ist die Menge an Augentropfen, die zur Verfügung steht, im Vergleich zu der abgenommenen Menge Blut gering, so dass mehrere Blutentnahmen notwedig sind, um Patienten mit einer ausreichenden Menge an Augentropfen über einen längeren Zeitraum zu versorgen.

Bevorzugt ist das Verabreichungsbehältnis ein Pumpsystem, welches ein Behältnis zur Aufnahme der Augentropfen sowie eine Pumpvorrichtung, zur Dosierung der Augentropfen in das Auge umfasst. Das Pumpsystem ist derart ausgestaltet, dass es sich zur Mehrfachverwendung eignet. Die Menge der darin enthaltenen Augentropfen ist dabei beispielsweise ausreichend, um eine Versorgung mit Augentropfen über einen Zeitraum von einer Woche oder länger zu ermöglichen. Deutlich längere Anwendungsdauern je Gefäß sind dabei auf Grund der Gefahr der Kontamination nicht gewünscht.

Die Menge an entnommenem Serum reicht dabei aus, eine Befüllung mehrerer Pumpsystem zu ermöglichen. Ein Pumpsystem wird dann an den Patienten abgegeben, die weiteren werden vorzugsweise am zweiten Ort, also insbesondere in der Apotheke, unter Kühlung gelagert. Bei Bedarf werden die Augentropfen dann an den Patienten ausgegeben. Da die Apotheke, also der zweite Ort, möglichst Wohnortnah ist, wird die Compliance hierdurch nicht verringert. Gleichzeitig erfolgt jedoch eine notwendige fachgerechte Lagerung der Augentropfen über einen längeren Zeitraum hinweg. Bei beziehungsweise vor jeder Ausgabe der Augentropfen erfolgt eine Kontrolle in der Apotheke, ob die Augentropfen noch eingesetzt werden können und der Patient kann rechtzeitig auf die Notwendigkeit einer neuen Blutentnahme hingewiesen werden, so dass eine konstante Versorgung mit Augentropfen und auch deren Kontrolle sicher gestellt ist, ohne dass dies mit einem großen Aufwand für den Patienten, also den Verwender der Augentropfen, verbunden ist, da der zweite Ort wohnortnah oder an einem anderen geeigneten Ort, wie beispielsweise in der Nähe des Arbeitsplatzes, gewählt werden kann.

Besonders bevorzugt sind Pumpsysteme, die derart ausgestaltet sind, dass eine mikrobielle Kontamination der Augentropfen während der Verwendung im Wesentlichen vermieden wird. Dies kann durch die Ausgestaltung der Pumpvorrichtung erfolgen, in welcher beispielsweise eine Membran enthalten sein kann, durch welche die Augentropfen nach außen gelangen können, Erreger jedoch nicht in das Innere des Pumpsystems. Alternativ kann die Pumpvorrichtung auch eine antibakterielle Vorrichtung, wie beispielsweise eine Silberspirale, umfassen. Entsprechende Pumpsysteme sind beispielsweise in EP 1 380 351 A1 beschrieben.

In einer bevorzugten Ausführungsform umfasst das Verfahren daher die folgenden Schritte:
a) Entnahme von Vollblut in einem Blutbeutel an einem ersten Ort und anschließender Transport des Vollblutes zu einem zweiten Ort,
b) Auftrennung des Blutes zum Erhalt von Serum an dem zweiten Ort,
   b1) Überführung des Serums unter sterilen Bedingungen in einen Serumbeutel an dem zweiten Ort,
c) Untersuchung des Serums auf das Vorliegen von Pathogenen am zweiten Ort und
d) Abfüllung des Serums in wenigstens ein Verabreichungsmittel am zweiten Ort, wobei das Verabreichungsmittel ein Pumpsystem ist, welches ein Behältnis zur Aufnahme der Augentropfen sowie eine Pumpvorrichtung, zur Dosierung der Augentropfen in das Auge umfasst.

Erfindungsgemäß erfolgt die Abfüllung des Serums dadurch, dass vorzugsweise in einem Reinraum unter sterilen Bedingungen mittels einer sterilen Spritze Serum aus dem Serumbeutel entnommen und die das Mehrdosen-Tropfsystem (Dosierflasche) mit Pumpsystem mit jeweils 5 ml befüllt wird. Die Aufsätze der Dosierflaschen werden manuell aufgesetzt, eine Hülse zum Schutz gegen Aufbrechen der Flaschen zugefügt und durch Bedienen einer Hebepresse fest mit der Flasche verschlossen. Der in der Dosierflasche beziehungsweise dem Pumpsystem enthaltende Filter schützt das Serum vor mikrobiologischer Kontamination. Eine Silberspirale in der Spitze verhindert das Wachstum von Keimen. Hierdurch ist das Totvolumen, also die Menge an Serum, die verworfen wird und nicht als Augentropfen zur Verfügung steht sehr gering, wodurch die Anzahl der Blutentnahmen gering gehalten werden kann.

Ein entsprechendes Pumpsystem ermöglicht eine definierte Einzeldosierung in Folge aus demselben Behälter. Gegenüber anderen Verabreichungsbehältnissen besteht hier der Vorteil darin, dass die Eigenserum Augentropfen auch über einen längeren Zeitraum von wenigstens 7 Tagen hinweg beim Patienten im Kühlschrank, also bei einer Temperatur von +2°C bis +8°C, oder sogar bei Raumtemperatur gelagert werden können, ohne dass eine Kontamination mit aeroben oder anaeroben Bakterien oder Pilzen auftritt. Durch den Kontakt mit der Silberspiral bei der Dosierung der Augentropfen werden diese im Wesentlichen frei von Mikroben gehalten. Gelichzeitig wird die Wirksamkeit der Augentropfen nicht beeinflusst wird.

Erfindungsgemäß ist es daher empfehlenswert, dass das Serum nach Abfüllung in geeignete Verabreichungsbehältnisse bei einer Temperatur von T ≤ -20°C gelagert wird. Hier ist eine Lagerung von wenigstens 6 Monaten möglich, ohne dass es zu einer Kontamination der Augentropfen kommt. Werden die so tiefgekühlt gelagerten Augentropfen aufgetaut und bei einer Temperatur von +2°C bis +8°C oder bei Raumtemperatur gelagert, sind diese wenigstens 7 Tage oder länger ohne eine Kontamination haltbar und damit vom Patienten einsetzbar. Auf Grund des erfindungsgemäßen Verfahrens ist es möglich, den Patienten wohnortnah wöchentlich mit neuen Augentropfen zu versorgen. So ist sichergestellt, dass die Lagerung bei -20 °C in einer Apotheke temperaturkontrolliert erfolgt. Einzelne Dosen, welche in einem haushaltsüblichen Kühlschrank, also bei einer Temperatur von +4°C bis +8°C, beziehungsweise bei Raumtemperatur wenigstens 7 Tage haltbar sind, können dann bei Bedarf übergeben werden. Somit können auch solche Personen über einen langen Zeitraum mit Serumaugentropfen versorgt werden, bei denen eine Blutentnahme beispielsweise auf Grund gesundheitlicher Probleme nicht oder nur selten möglich ist.

Erfindungsgemäß ist es möglich, dass zur Herstellung der Augentropfen das Serum vor oder während der Abfüllung in geeignete Verabreichungsbehältnisse verdünnt wird. Diese Verdünnung kann beispielsweise mittels bekannter künstlicher Tränenersatzmittel erfolgen, welche beispielsweise isotonische Kochsalzlösung und/oder Wirkstoffe wie Hyaluronsäure und/oder Lipide umfassen. Dabei kann die Verdünnung im Bereich von 10% bis 30% liegen. Dies bedeutet im Sinne der vorliegenden Erfindung, dass bezogen auf 100 Gew.-% an Augentropfen diese zu 90 Gew.-% aus Serum und zu 10 Gew.-% aus Tränenersatzmittel bestehen. Entsprechend bedeutet eine Verdünnung von 20 Gew.-%, dass die Augentropfen aus 80 Gew.-% Serum und 20 Gew.-% Tränenersatzmittel bestehen, und eine Verdünnung von 30 Gew.-%, dass die Augentropfen aus 70 Gew.-% Serum und 30 Gew.-% Tränenersatzmittel bestehen.

Die Verdünnung der Serumaugentropfen ermöglicht eine Versorgung von betroffenen Personen über einen Zeitraum von annähernd einem Jahr beziehungsweise je nach Häufigkeit der Anwendung auch darüber hinaus, wodurch sich ebenfalls eine verbesserte Compliance ergibt. Darüber hinaus hat sich überraschenderweise gezeigt, dass bei dem Vorliegen von Läsionen der Augenhornhaut, wie sie beispielweise bei einer Leukämieerkrankung auftreten, es zu Proteinablagerungen im Auge kommen kann, wenn unverdünnte Serumaugentropfen eingesetzt werden. Durch eine erfindungsgemäße Verdünnung können solche Proteinablagerungen vermieden werden.

Weiterhin ermöglicht die Verdünnung eine lange Versorgung von Patienten insbesondere auch dann, wenn diese auf Grund einer Erkrankung nicht erneut zur Blutabnahme gehen können. Dies kann beispielsweise der Fall bei einer Leukämieerkrankung sein, bei welcher zur Therapie Stammzellen transplantiert wurden. Betroffene Patienten haben das Risiko, dass sich bedingt durch eine Immunreaktion gegen die transplantierten Stammzellen eine "graft versus host desease" entwickelt, bei welcher es bei den Erkrankten zu den bereits beschriebenen Hornhautläsionen im Auge kommen kann. Diese können ebenfalls mit Serumaugentropfen gut behandelt werden. Allerdings kann hier auf Grund der Erkrankung eine regelmäßige Blutentnahme nicht sichergestellt werden. Das erfindungsgemäße Verfahren ermöglicht hier Abhilfe.

Die Verdünnung sollte einen Wert von 30 % jedoch nicht überschreiten. Bei einer stärkeren Verdünnung verändern sich die Fließeigenschaften der Augentropfen, so dass eine gute Anhaftung am Auge nicht mehr gegeben ist. Zudem werden auch die weiteren Eigenschaften, wie beispielsweise die Proteinkonzentration, derart beeinflusst, dass die positiven Eigenschaften der Serumaugentropfen gegenüber konventionellen Tränenersatzmitteln kaum überwiegen.

Neben der Untersuchung des Serums auf das Vorliegen von Krankheiten und/oder Infektionen vor der Abfüllung des Serums ist es erfindungsgemäß weiterhin bevorzugt, dass auch nach der Abfüllung des Serums in Verabreichungsbehältnisse stichprobenartig wenigstens zwei Verabreichungsbehältnisse herausgesucht werden und das darin enthaltende Serum nochmals auf das Vorliegen von Krankheiten und/oder Infektionen untersucht wird. Da erfindungsgemäß eine größere Menge Blut zu Beginn entnommen wird, steht eine ausreichende Menge Serum zur Verfügung, um einerseits dem Patienten mit Augentropfen über einen Zeitraum von wenigstens einem halben Jahr zu versorgen und auf der anderen Seite durch eine ausreichende Anzahl von Stichproben zu gewährleisten, dass auch bei der Abfüllung in Schritt d) des erfindungsgemäßen Verfahrens keine weitere Kontamination erfolgt ist.

In einer weiteren Ausführungsform betrifft die vorliegende Erfindung weiterhin Augentropfen, welche nach dem erfindungsgemäßen Verfahren hergestellt wurden. Durch die erfindungsgemäß hergestellten Augentropfen ist eine wohnortnahe, langfristige Versorgung sichergestellt. Auf Grund der einmaligen Blutspende sowie einer verbesserten Sicherheit vor Infektionen, auf Grund der Möglichkeit zur Testung des Serums, ergibt sich eine verbesserte Compliance und damit erhöhte Chancen auf eine erfolgreiche Behandlung von trockenen Augen.

Erfindungsgemäß können die Augentropfen zur Behandlung des Krankheitsbildes des trockenen Auges verwendet werden. Somit können beispielsweise Patienten, bei welchen trockene Augen neben der eigentlichen Erkrankung, wie beispielsweise Diabetes oder Rheuma, entsprechende Augentropfen einsetzen. Weiterhin eignen sich die erfindungsgemäßen Augentropfen zur Behandlung von trockenen Augen, welche als Nebenwirkungen von Medikamenten, wie beispielsweise Hormonpräparate, Betablocker oder Psychopharmaka, auftreten. Auch Läsionen der Hornhaut am Auge, wie sie durch Chemotherapeutika verursacht werden können, können mit den erfindungsgemäßen Augentropfen behandelt werden. Dabei eignen sich die Augentropfen nicht nur bei krankheitsbedingten trockenen Augen, sondern können auch von Menschen, die sehr viel am Computer arbeiten oder auf Grund des Arbeitsplatzes in besonders trockenen, kalten Räumen verwendet werden.

Weiterhin betrifft die vorliegende Erfindung ein Kit, welches die Augentropfen in einem geeigneten Verabreichungsbehältnis umfasst.

Geeignete Verabreichungsbehältnisse sind aus dem Stand der Technik bekannt und ermöglichen eine gleichbleibende Dosierung der Augentropfen. Bevorzugt ist ein Verabreichungsbehältnis, welches ein Pumpsystem, welches ein Behältnis zur Aufnahme der Augentropfen sowie eine Pumpvorrichtung zur Dosierung der Augentropfen aufweist, umfasst, da hier die Menge an Augentropfen, welche bei der Verwendung dosiert wird, gleichbleibend ist.

### Ausführungsbeispiele:

### Ausführungsbeispiel 1:

Zur Untersuchung der Fließeigenschaften sowie der Keimfreiheit von Serumaugentropfen wurden im Rahmen einer Vollblutspende 5 Personen jeweils 20 ml Blut entnommen. Die Blutentnahme erfolgte mittels ACD-freier Blutentnahmeröhrchen. Entsprechende Blutentnahmeröhrchen weisen keine Koagulationshemmer (ACD: Acid-Citrate-Dextrose) auf, so dass das Blut im erfindungsgemäßen Verfahren eingesetzt werden kann. Nach der Blutentnahme erfolgte die Koagulation bei Raumtemperatur in stehenden Röhrchen. Die Koagulation war vollständig, wenn der Blutkuchen, der die festen Bestandteile des Blutes umfasst, mit einem Spatel von der Gefäßwand gelöst werden konnte. Dies konnte nach 20 bis 30 Minuten erfolgen.

Nach der Entnahme des Blutkuchens aus den Röhrchen wurde das darin enthaltene Serum bei 3500 Umdrehungen/min für 20 min zentrifugiert. Unmittelbar anschließend wurde der Serumüberstand abpipettiert und das so erhaltene Serum im Kühlschrank bei einer Temperatur von 4°C bis 8°C gelagert.

Das Serum wurde in Verabreichungsgefäße mit Pumpsystem, wie sie von der Firma Aero Pump GmbH als Augentropfer-System mit 3K^{®}-System vertrieben werden, abgefüllt. Es wurden die folgenden gebrauchsfertigen Augentropfen erhalten:
- Beispiel AT 1:: 5ml Serum im Verabreichungsgefäß
- Beispiel AT 2:: 5ml Serumaugentropfen (10 % Verdünnung mit isotonischer Kochsalzlösung)
- Beispiel AT 3:: 5ml Serumaugentropfen (20 % Verdünnung mit isotonischer Kochsalzlösung)
- Beispiel AT 4:: 5ml Serumaugentropfen (30 % Verdünnung mit isotonischer Kochsalzlösung)

Eine Verdünnung von 10 % bedeutet, dass 90 Gew.-% der Serumaugentropfen Serum und 10 Gew.-% isotonische Kochsalzlösung sind. Entsprechendes gilt für die anderen Verdünnungen.

Die Aufbewahrung der gebrauchsfertigen Augentropfen erfolgte im Kühlschrank bei einer Temperatur von 4°C bis 8°C. Es wurden 5 mal täglich über einen Zeitraum von 9 Tagen hinweg Proben aus den jeweiligen Augentropfen AT1 bis AT 4 entnommen.

Alle Proben wiesen eine sehr gute Fließfähigkeit und eine leichte Rosafärbung, die mit zunehmender Verdünnung abnahm, auf. Die entnommenen Proben wiesen durchgehend das gleiche Volumen auf. Auch nach 9 Tagen war keine mikrobielle Verunreinigung der Augentropfen sichtbar.

### Ausführungsbeispiel 2:

Vollblut (500 ml) wurde von 10 Personen nach Standardverfahren des DRK-Blutspendedienstes (Standard Operation Procedure) in Blutbeutel entnommen. Nach der Koagulation des Blutes (über Nacht bei Raumtemperatur) und Zentrifugation (15 Minuten bei 3000 Umdrehungen/min) wurde das Serum in an den Blutbeutel angeschweißte Leerbeutel überführt.

Das Serum wurde bei + 4 °C in einen Reinraum (Klasse A in B) transportiert. Dort erfolgte die Abfüllung des Serums in Verabreichungsgefäße mit Pumpsystem, wie sie von der Firma Aero Pump GmbH als Augentropfer-System mit 3K^{®}-System vertrieben werden, abgefüllt. Die Proben wurden jeweils unterschiedlich gelagert und vor der Abfüllung und danach auf ihre Keimfreiheit untersucht:
Beispiel AT 5: Serum vor der Abfüllung in das Verabreichungsgefäß
Beispiel AT 6: Serum in Pumpsystem nach 48 h Lagerung bei - 20°C
Beispiel AT 7: Serum in Pumpsystem nach 48 h Lagerung bei +4 °C bis +8 °C
Beispiel AT 8: Serum in Pumpsystem nach 48 h Lagerung bei Raumtemperatur (20 °C)
Beispiel AT 9: Serum in Pumpsystem nach 5 Tagen Lagerung bei Raumtemperatur (20 °C)

In allen Beispielen AT 5 bis AT 9 waren die durchgeführten Sterilkontrollen (Testungen gemäß Europäischem Arzneimittelbuch auf aerobe und anaerobe Keime und Pilze) negativ. Die Kontrollen erfolgten mit einem automatisierten Nachweisverfahren BacT/Alert von Bio Merieux, Nürtingen.

## Patentansprüche

1. Verfahren zur Herstellung von gebrauchsfertigen Augentropfen umfassend die folgenden Schritte:
a) Entnahme von Vollblut an einem ersten Ort und anschließende
b) Auftrennung des Blutes zum Erhalt von Serum,
c) Untersuchung des Serums auf das Vorliegen von Pathogenen gefolgt von
d) Abfüllung des Serums in wenigstens ein Verabreichungsmittel an einem zweiten Ort,
wobei erster Ort und zweiter Ort voneinander verschieden sind.

2. Verfahren nach Anspruch 1, weiterhin umfassend Schritt
e) Abschlussuntersuchungen vor der Freigabe der Augentropfen als Arzneimittel Sterilität.

3. Verfahren nach Anspruch 1 oder 2, **dadurch gekennzeichnet, dass** das Vollblut autolog oder allogen ist.

4. Verfahren nach einem der Ansprüche 1 bis 3, **dadurch gekennzeichnet, dass** die Abfüllung in Schritt d) in einem Reinraum, insbesondere in einem Reinraum in einer Apotheke erfolgt.

5. Verfahren nach einem der Ansprüche 1 bis 4 umfassend die folgenden Schritte:
a) Entnahme von Vollblut in einem Blutbeutel an einem ersten Ort und anschließender Transport des Vollblutes zu einem zweiten Ort,
b) Auftrennung des Blutes zum Erhalt von Serum an dem zweiten Ort,
b1) Überführung des Serums unter sterilen Bedingungen in einen Serumbeutel an dem zweiten Ort,
c) Untersuchung des Serums auf das Vorliegen von Pathogenen am zweiten Ort und
d) Abfüllung des Serums in wenigstens ein Verabreichungsmittel am zweiten Ort, wobei das Verabreichungsmittel ein Pumpsystem ist, welches ein Behältnis zur Aufnahme der Augentropfen sowie eine Pumpvorrichtung, zur Dosierung der Augentropfen in das Auge umfasst.

6. Verfahren nach einem der Ansprüche 1 bis 5, **dadurch gekennzeichnet, dass** das Serum vor oder während des Abfüllens in Schritt d) verdünnt wird.

7. Verfahren nach Anspruch 6, **dadurch gekennzeichnet, dass** die Verdünnung im Bereich von 10% bis 30% erfolgt.

8. Verfahren nach Anspruch 6 oder 7, **dadurch gekennzeichnet. dass** die Verdünnung mit bekannten Tränenersatzmitteln, insbesondere mit isotoner Kochsalzlösung erfolgt.

9. Augentropfen erhältlich nach dem Verfahren gemäß einem der Ansprüche 1 bis 8.

10. Verwendung von Augentropfen gemäß Anspruch 9 zur Behandlung des trockenen Auges.

11. Kit umfassend Augentropfen gemäß Anspruch 9 in einem Verabreichungsbehältnis.

12. Kit nach Anspruch 11, **dadurch gekennzeichnet, dass** das Verabreichungsbehältnis ein Pumpsystem, welches ein Behältnis zur Aufnahme der Augentropfen sowie eine Pumpvorrichtung zur Dosierung der Augentropfen aufweist, umfasst.
